# FASCICULE DE BREVET EUROPEEN

(11) **EP 3 887 011 B1**
(45) Date de publication et mention de la délivrance du brevet: **03.04.2024**
(21) Numéro de dépôt: 19813455.3
(22) Date de dépôt: 27.11.2019
(51) Int. Cl.: B01D 11/02, A61K 8/06, A23L 33/105, A61K 36/00, A61K 8/9789, A61K 36/56

(54) **PROCÉDÉ D'EXTRACTION DE SUBSTANCES D'INTÉRÊT**
VERFAHREN ZUR EXTRAKTION VON SUBSTANZEN VON INTERESSE
METHOD FOR EXTRACTING SUBSTANCES OF INTEREST

(30) Priorité: 27.11.2018 FR 1871961; 19.03.2019 FR 1902826
(43) Date de publication de la demande: 06.10.2021
(73) Titulaire: Avignon Universite, 84029 Avignon Cedex 1 (FR); LYOFAL, 13300 Salon-de-Province (FR); Centre national de la recherche scientifique, 75016 Paris (FR); Ecole Nationale Supérieure de Chimie de Montpellier, 34296 Montpellier Cedex 5 (FR); Université de Montpellier, 34090 Montpellier (FR)
(72) Inventeur: CONTINO-PEPIN, Christiane, 84210 ALTHEN des PALUDS (FR); DESGRANGES, Stéphane, 84000 AVIGNON (FR); DALL'ARMELLINA, Alice, 84000 AVIGNON (FR); DUVAL, Charles, 13430 EYGUIERES (FR); LETAN-MARTIN, Mathias, 84440 ROBION (FR)
(74) Mandataire: Osha BWB
(86) Numéro de dépôt international: PCT/EP2019/082797
(87) Numéro de publication internationale: WO 2020/109418

(56) Documents cités:
- CN-B- 101 978 996
- FR-A1- 2 903 016
- KR-A- 20140 115 427
- KR-A- 20150 107 504
- NGUYEN H T P ET AL: "Novel alginate-based nanocarriers as a strategy to include high concentrations of hydrophobic compounds in hydrogels for topical application", NANOTECHNOLOGY, INSTITUTE OF PHYSICS PUBLISHING, GB, vol. 26, no. 25, 2 juin 2015 (2015-06-02), page 255101, XP020286452, ISSN: 0957-4484, DOI: 10.1088/0957-4484/26/25/255101 [extrait le 2015-06-02]
- DIMA STEFAN ET AL: "Encapsulation of Functional Lipophilic Food and Drug Biocomponents", FOOD ENGINEERING REVIEWS, SPRINGER, US, vol. 7, no. 4, 4 mars 2015 (2015-03-04), pages 417-438, XP035580094, ISSN: 1866-7910, DOI: 10.1007/S12393-015-9115-1 [extrait le 2015-03-04]

## Description

### Domaine technique de l'invention

La présente description concerne un procédé d'extraction d'au moins une substance d'intérêt ainsi que des émulsions produites par un tel procédé, ou encore des utilisations de telles émulsions. La présente description trouve des applications notamment mais pas exclusivement dans les domaines agroalimentaire, cosmétique, pharmaceutique, ou encore dans le domaine des parfums.

### Etat de la technique

Les substances naturelles, telles que par exemple celles d'origine végétale, animale, ou minérale, connaissent un intérêt croissant dans de nombreux domaines. En effet, compte tenu de la grande chimio-diversité moléculaire qu'offre, par exemple, le règne végétal et de l'engouement du grand public pour les produits naturels, un certain nombre de secteurs industriels (cosmétique, pharmaceutique, agroalimentaire, nutraceutique, probiotique) se tournent vers l'incorporation de molécules d'origine végétale dans leurs formulations. La valorisation de ces principes actifs d'origine naturelle représente donc un potentiel économique notable pour le vivier d'entreprises des secteurs agroalimentaire, cosmétique et pharmaceutique.

Pour être valorisés, les composés naturels tels que des composés bioactifs, c'est-à-dire des composés présentant une activité biologique, peuvent d'abord être séparés de leur matrice végétale au cours d'une étape d'extraction, avant d'être utilisés tels quels, sous forme d'extrait, ou après purification sous forme de molécules isolées. Le secteur de l'extraction est particulièrement concerné par les enjeux environnementaux et économiques et tente aujourd'hui de trouver des solutions afin de réduire les dépenses énergétiques et les rejets tout au long de la chaîne des procédés d'extraction.

Dans le domaine de l'extraction des produits naturels à haute valeur ajoutée, l'extraction par solvant reste la méthode la plus employée. Cependant, cette extraction fait appel à des solvants organiques tels que par exemple l'hexane, le cyclohexane, l'éther de pétrole, le méthanol, l'éthanol ou à l'utilisation de mélanges hydro-alcooliques. Par exemple, si le produit naturel à extraire, i.e. la substance d'intérêt, est hydrophile, le solvant d'extraction utilisé pourra être polaire aprotique tel que l'acétone, ou polaire protique tel que l'eau, le méthanol ou l'éthanol. Si en revanche le produit naturel à extraire, i.e. la substance d'intérêt, est lipophile, le solvant d'extraction utilisé pourra alors être apolaire, i.e. hydrophobe, tel que l'hexane, ou moins apolaire que l'hexane, tel que le chloroforme ou le dichlorométhane. Ces solvants sont pour bon nombre d'entre eux inflammables, volatiles et toxiques. Par ailleurs, l'épuisement progressif des ressources pétrolières et surtout le durcissement de la réglementation obligent les industriels à s'orienter vers des méthodes plus respectueuses de l'environnement. La recherche d'alternatives à l'utilisation des solvants organiques et/ou de procédés d'extraction consommateurs d'énergie, parfois dénaturants pour les biomolécules, est un enjeu à la fois économique et scientifique majeur auquel la présente invention permet de répondre. Malgré le caractère ubiquitaire de l'eau chez le végétal, de nombreuses molécules bioactives d'origine végétale (telles que les vitamines, les antioxydants, les antiinflammatoires naturels...) sont peu voire insolubles dans l'eau, ce qui limite les rendements d'extraction et la qualité (par exemple en termes d'intégrité et de stabilité) des extraits produits. Des exemples des procédés d'extraction se trouvent dans NGUYEN HTP et al, DIMA et al et KR20150107504.

La présente description a entre autres pour objet un procédé, qui, dans certaines formes de réalisation, peut permettre d'extraire, sans les altérer et avec de bons rendements, des substances d'intérêt, par exemple liposolubles, d'origine végétale, animale, ou minérale par émulsification, e.g., par sonification, d'un milieu aqueux additionné d'un composé lipophile et d'un tensioactif synthétique ou naturel, et d'aboutir dans une même étape (« one-pot »), à une formulation stable dans le temps, facile à stocker et à transporter.

### Résumé de l'invention

Dans la présente description et dans les revendications qui suivent, le terme « comprendre » est synonyme de (signifie la même chose que) « inclure », « contenir », et est inclusif ou ouvert et n'exclut pas d'autres éléments non décrits ou représentés. En outre, dans la présente description, le terme « environ » est synonyme de (signifie la même chose que) une marge inférieure et/ou supérieure de 10%, par exemple 5%, de la valeur respective.

Dans la présente description et dans les revendications, par D, on entend le diamètre moyen des gouttelettes, mesuré avec un granulomètre, basé sur la diffraction de la lumière, pour les gouttelettes de diamètre D supérieur à 1000 nm (diamètre moyen de De Brouckère en volume-poids des particules de l'émulsion), et avec un appareil de Diffusion Dynamique de la Lumière (DLS) pour les gouttelettes de diamètre D inférieur à 1000 nm. Lorsque l'appareil de Diffusion Dynamique de la Lumière (DLS) est utilisé pour mesurer D, par indice de polydispersité (ou PdI) d'une émulsion, on entend le ratio du carré de l'écart type sur le carré du diamètre moyen des gouttelettes. Lorsque le granulomètre est utilisé, par indice de polydispersité (ou Pdl) d'une émulsion, on entend le rapport D90/D10, où par diamètre D10 des gouttelettes, on entend le diamètre de gouttelette pour lequel 90% de la population totale des gouttelettes de l'émulsion ont un diamètre supérieur ou égal à cette valeur, et par D90, on entend le diamètre de gouttelette pour lequel 10 % de la population totale de gouttelettes de l'émulsion ont un diamètre supérieur à cette valeur.

Un des objectifs de la présente description est de fournir un procédé d'extraction d'au moins une substance d'intérêt à partir d'une source. L'au moins une substance d'intérêt peut soit être contenue dans la source, soit être générée par ledit procédé d'extraction dans le cas où l'au moins substance d'intérêt dérive d'au moins une molécule préalablement contenue dans ladite source, qui, lors du procédé d'extraction, est chimiquement transformée en ladite au moins une substance d'intérêt.

Selon un premier aspect, la présente description concerne un procédé d'extraction d'au moins une substance d'intérêt à partir d'une source, le procédé comprenant les étapes suivantes :
- la fourniture d'un mélange comprenant de l'eau, une source végétale, animale, minérale, procaryote, et/ou eucaryote unicellulaire comprenant l'au moins une substance d'intérêt ou au moins un précurseur de celle-ci, au moins un tensioactif, et au moins un composé lipophile;
- l'émulsification dudit mélange, conduisant à la formation d'une émulsion ;

et dans lequel ladite émulsion comprend :
   - une phase aqueuse continue; et
   - une phase lipidique discontinue comprenant l'au moins un composé lipophile, ledit au moins un tensioactif et l'au moins une substance d'intérêt extraite de ladite source durant l'émulsification;
dans lequel le procédé comprend en outre l'ajout d'au moins un cryoprotecteur au mélange et la lyophilisation de l'émulsion ; et
dans lequel l'ajout d'au moins un cryoprotecteur au mélange est effectué après l'émulsification du mélange, dans un rapport poids-volume compris entre 5 % p/v et 10 % p/v par rapport au volume total du mélange excluant la source.

Le procédé selon des formes de réalisation de la présente description peut avantageusement être « one pot », sans altérer l'au moins une substance d'intérêt et avec de bons rendements en substance d'intérêt. L'extraction de molécules par émulsification, dans un milieu aqueux additionné de composé lipophile et de tensioactif répond à une démarche de « chimie verte » et de respect de l'environnement.

Selon une ou plusieurs formes de réalisation, l'émulsification du mélange comprend l'application d'une source d'énergie au mélange, pouvant être une énergie ultrasonore et/ou une énergie purement mécanique. Selon une ou plusieurs formes de réalisation, l'émulsification du mélange comprend la sonification du mélange. La sonification du mélange comprend l'application d'une source d'énergie ultrasonore au mélange, conduisant à la formation d'une émulsion.

Ainsi, le procédé comprend l'émulsification d'un mélange comprenant de l'eau, une source comprenant l'au moins une substance d'intérêt ou au moins un précurseur de celle-ci, au moins un tensioactif, et au moins un composé lipophile. L'émulsification de ce mélange conduit à la formation d'une émulsion comprenant une phase aqueuse continue et une phase lipidique discontinue comprenant l'au moins un composé lipophile, ledit au moins un tensioactif et au moins une substance d'intérêt extraite de ladite source, durant l'émulsification. L'émulsification induit la formation d'une émulsion, ce qui a pour effet d'augmenter la surface de contact entre les deux phases aqueuse et lipidique, et ainsi le transfert de l'au moins une substance d'intérêt d'une phase à l'autre. Selon une ou plusieurs formes de réalisation, après l'émulsification du mélange conduisant à la formation de l'émulsion, une partie résiduelle du tensioactif peut être comprise dans la phase aqueuse.

Selon un aspect, la présente description concerne un procédé d'extraction d'au moins une substance d'intérêt à partir d'une source, le procédé comprenant les étapes suivantes :
- la fourniture d'un mélange comprenant de l'eau, une source végétale, animale, minérale, procaryote, et/ou eucaryote unicellulaire comprenant l'au moins une substance d'intérêt ou au moins un précurseur de celle-ci, au moins un tensioactif, et au moins un composé lipophile;
- la sonification dudit mélange, conduisant à la formation d'une émulsion ;

et dans lequel ladite émulsion comprend :
   - une phase aqueuse continue; et
   - une phase lipidique discontinue comprenant l'au moins un composé lipophile, ledit au moins un tensioactif et l'au moins une substance d'intérêt extraite de ladite source durant la sonification ;
dans lequel le procédé comprend en outre l'ajout d'au moins un cryoprotecteur au mélange et la lyophilisation de l'émulsion ; et
dans lequel l'ajout d'au moins un cryoprotecteur au mélange est effectué après l'émulsification du mélange, dans un rapport poids-volume compris entre 5 % p/v et 10 % p/v par rapport au volume total du mélange excluant la source.

En jouant sur le caractère lipophile de certaines molécules difficiles à extraire dans l'eau pure (e. g., sans ajout d'alcool), les inventeurs ont pu aboutir à un procédé d'extraction dans l'eau additionnée d'au moins un composé lipophile et de tensioactifs d'origine naturelle ou synthétique, permettant d'aboutir, en un nombre limité d'étapes, à des extraits de type «huile et/ou cire dans eau», enrichis en molécules d'intérêt, potentiellement bioactives.

La source peut être par exemple végétale (telle que le marc d'olive, eaux de végétation, résidus de vinification, etc.), animale (telle que carcasses, peaux, ou d'autres parties des animaux), minérale, procaryote, et/ou eucaryote unicellulaire. Selon une ou plusieurs formes de réalisation, la source peut provenir de sous-produits de l'industrie alimentaire et/ou agricole, qu'elle soit végétale, animale, minérale, procaryote, et/ou eucaryote unicellulaire ou autre. La source peut être constituée par n'importe quelle matière susceptible de contenir ou de donner lieu à au moins une substance d'intérêt. Une substance d'intérêt peut par exemple comprendre une molécule lipophile et peu soluble dans l'eau (liposoluble), et potentiellement intéressante pour une application dans le domaine cosmétique, pharmaceutique, et/ou agroalimentaire. Ladite molécule peut par exemple présenter une activité biologique ayant un impact bénéfique sur la santé humaine. Les antioxydants par exemple, sont des molécules pouvant intervenir de façon prépondérante dans la protection de l'organisme contre le développement des maladies cardiovasculaires et de certains cancers, notamment du fait de leur aptitude à piéger rapidement les espèces oxygénées réactives impliquées dans le développement de ces maladies. Ladite molécule lipophile peut par exemple comprendre un polyphénol ou un terpène. Selon une ou plusieurs formes de réalisation, la source est végétale. La source végétale peut contenir au moins une partie d'au moins une plante (graine, feuille, racine, tige, fruit, fleur, etc.). La source végétale peut être citée, par exemple, dans l'ouvrage d'Albert Y. Leung et Steven Foster, "Encyclopedia of common natural ingrédients used in food, drugs and cosmetics", 2ème édition, Wiley-Interscience, 1996. La source végétale peut par exemple être choisie parmi les sources végétales associées à des propriétés adoucissantes, des propriétés anti-inflammatoires, des propriétés antiseptiques, des propriétés antisudorales, des propriétés calmantes, des propriétés cicatrisantes, des propriétés tonifiantes, des propriétés favorisant la contention de la microcirculation sanguine et lymphatique, des propriétés texturantes, des propriétés antioxydantes, des propriétés moussantes ou émulsifiantes, des propriétés photoprotectrices, des propriétés épaississantes, absorbantes et/ou des propriétés odorantes.

A titre d'exemples de sources végétales classées en fonction de leurs différentes propriétés, on peut notamment citer :
pour leurs propriétés adoucissantes : l'abricotier, le bleuet, le bouillon blanc, la camomille romaine, la matricaire, le coquelicot, le fenugrec, la guimauve, le lin, le lis, la mauve, le souci, le sureau, le tilleul, le tussilage, le psyllium, le plantain, le cognassier, le pêcher, l'oranger, le cactus, le pommier ;
pour leurs propriétés anti-inflammatoires : l'aigremoine, l'aubépine, la bruyère, le chiendent, le genévrier, la guimauve, le sureau, le tilleul, le fenugrec, la gentiane, la laitue, la pensée sauvage, le plantain, la ronce, le romarin, la sauge, le tamier, le tussilage, l'immortelle, la pâquerette ;
pour leurs propriétés antiseptiques : l'ail, l'aigremoine, la myrtille, la bardane, le chêne, la consoude, l'eucalyptus, le genévrier, le géranium rose, le laurier, la lavande, la marjolaine, la menthe, le pin, le romarin, le santal, le serpolet, le thym, la sauge, le chèvrefeuille, l'immortelle, la pâquerette, la tanaisie, le piment, le poivre ;
pour leurs propriétés antisudorales : la sauge, le chêne, le noyer, le pin, la prêle, le tussilage ;
pour leurs propriétés astringentes : l'acacia, l'achillée millefeuille, l'aigremoine, l'alchémille, l'arbousier, l'armoise, la consoude, le cyprès, le chêne, l'églantier, l'hamamélis, le mûrier noir, la myrtille, le noisetier, le noyer, l'ortie, le peuplier, le plantain, la ronce, le ratanhia, le rosier, la salicaire, le saule, la tormentille, la vigne rouge, le mélilot ;
pour leurs propriétés calmantes : la carotte, la pensée sauvage, le sureau, le tilleul, le passiflore, le basilic, le camphre, le poirier, le pommier, la vigne, la laitue, le rosier, le gingembre ;
pour leurs propriétés cicatrisantes : le millepertuis, la potentille ansérine, le souci, la matricaire, la camomille romaine, la consoude, l'achillée millefeuille, l'absinthe, l'aigremoine, l'armoise, l'arnica, le cerfeuil, la myrte, la pervenche, le plantain, le peuplier, la primevère, la sauge, le séneçon, le sureau, la verveine, l'angélique, l'aristoloche, l'aulne, l'aurone, la bistorte, le bouleau, le chardon béni, le genévrier, le néflier, l'eucalyptus, la vulnéraire, la benoite, la centaurée, le chou, la joubarbe, le fraisier, la prêle, la reine des prés, le tussilage, la pensée sauvage, la bardane, la pâquerette, le lis ;
pour leurs propriétés tonifiantes : le millepertuis, l'églantine, le gui, le maté, le cassier, l'absinthe, l'arnica, le calament, la cannelle, le géranium, l'hysope, la marjolaine, la mélisse, le persil, le pin sylvestre, le romarin, la sarriette, le serpolet, le basilic, l'églantier, la gentiane, le houblon, le laurier blanc, la menthe, la sauge, la tormentille, l'achillée millefeuille, l'aigremoine, la benoite, la bistorte, le chêne, le cognassier, le cyprès, le marron d'Inde, le néflier, le noyer, l'ortie, le plantain, la poire d'eau, le quintefeuille, la renouée, la salicaire, la véronique, l'angélique, l'armoise, l'aspérule, le genévrier, la moutarde, le quinquina, le fumeterre, la capucine, le cresson, le varech, le fragon, la tanaisie ;
pour leurs propriétés favorisant la contention de la microcirculation sanguine et lymphatique : le cassis, la myrtille et les pépins de raisin ;
pour leurs propriétés texturantes : le blé, le fucus ;
pour leurs propriétés antioxydantes : le riz, le romarin, la sauge, le thym, le thé vert, le réglisse, le piment ;
pour leurs propriétés moussantes ou émulsifiantes : la saponaire, le lierre, le fragon, le bois de panama, le quillaja, la salsepareille, le quinoa, le soja ;
pour leurs propriétés photoprotectrices : l'aloès, le tournesol, la réglisse, le magnolia, le kaempferia ;
pour leurs propriétés épaississantes ou absorbantes : le pois, le blé, les pommes de terre, le maïs ; et
pour leurs propriétés odorantes : le romarin, la violette, la lavande, le jasmin, le muguet la vanille, la rose, les agrumes, e.g. le citron, le pamplemousse. Selon une ou plusieurs formes de réalisation, la source végétale est la plante *Curcuma longa.*

Selon une ou plusieurs formes de réalisation, la source comprend la propolis et/ou du miel.

L'au moins un composé lipophile peut être contenu dans la source et/ou résulter d'un ajout exogène préalable. En fonction de la source considérée, l'ajout exogène de l'au moins un composé lipophile ne sera pas toujours nécessaire pour obtenir une émulsion stable. Par exemple, certaines matrices végétales peuvent libérer sous l'effet des ultrasons des huiles essentielles, des phospholipides, des protéines amphiphiles. Par exemple, les écorces d'agrumes peuvent libérer sous l'effet des ultrasons une quantité variable d'huile essentielle qui peuvent suffire à l'obtention des émulsions visées par le procédé selon des formes de réalisation de la présente description. Selon une ou plusieurs formes de réalisation, l'au moins un composé lipophile est sélectionné dans le groupe comprenant les huiles, les cires, et combinaisons de celles-ci. L'au moins un composé lipophile peut par exemple comprendre un mélange entre plusieurs huiles, entre plusieurs cires, ou un mélange huile(s)/cire(s). Les inventeurs ont constaté que pour certaines sources, la présence d'au moins deux composés lipophiles dans le mélange fourni par le procédé de l'invention pouvait permettre de réduire l'indice de polydispersité de l'émulsion et/ou de diminuer le diamètre moyen D des gouttelettes de l'émulsion et/ou d'augmenter le taux d'extraction de l'au moins une substance active, e.g. en présence de deux huiles différentes, ou encore d'une huile et d'une cire, par opposition au cas où une seule huile est présente dans le mélange. Selon une ou plusieurs formes de réalisation, le composé lipophile est sélectionné dans le groupe comprenant les mono-, di- ou triesters de glycérol, ou dérivés du glycérol, les mono-, di- ou tri- ou tétra-esters de l'acide citrique ou dérivés de l'acide citrique, les acides gras, les monoesters d'acides gras, les huiles essentielles, les substituts de graisse, les cires, et combinaisons de ceux-ci. Avantageusement, le procédé selon la présente description ne nécessite pas d'utiliser un solvant organique d'extraction typique, tel que par exemple l'hexane, le cyclohexane, le dichlorométhane, le tétrahydrofurane, le diméthylsulfoxyde, l'éther de pétrole, le méthanol, l'éthanol ou un mélange hydro-alcoolique. Selon une ou plusieurs formes de réalisation, l'au moins un composé lipophile est présent dans le mélange, en excluant la source, dans un rapport poids-volume compris entre 0,01 % p/v et 70 % p/v, par exemple entre 3% p/v et 70 % p/v, par rapport au volume total du mélange comprenant l'eau, le composé lipophile et le tensioactif. Le rapport poids-volume de l'au moins un composé lipophile dans le même mélange, en excluant la source, est par exemple compris entre 0,1 % p/v et 20 % p/v, par exemple entre 5 % p/v et 20 % p/v.

L'au moins un tensioactif peut être anionique, cationique, zwittérionique, amphotère ou non-ionique. L'au moins un tensioactif peut être naturel ou synthétique. Selon une ou plusieurs formes de réalisation, son équilibre hydrophile-lipophile, ou HLB (pour Hydrophilic Lipophilic Balance), est compris entre 4 et 19. L'au moins un tensioactif peut par exemple être sélectionné dans le groupe comprenant des tensioactifs non ioniques tels que des alcools, aminoalcools, esters, amine-oxydes, et combinaisons de ceux-ci. Le tensioactif peut être par exemple le Lauroglycol^{™} FCC (Propylene glycol monolaurate (type I)), Lauroglycol^{™} 90, Capryol^{™} 90, Plurol^{®} Oleique CC 497 (Polyglyceryl-3 dioleate), Labrasol^{®}, Caprylocaproyl Polyoxyl-8 glycerides, Labrafil^{®} M 1944 CS, Labrasol^{®} ALF, Labrafil^{®} M 2125 CS , Gelucire^{®} 50/13, Labrafil^{®} M 2130 CS, Gelucire^{®} 48/16, Gelucire^{®} 44/14, Transcutol^{®} HP, Emulfree^{®} CBG MB. Le tensioactif peut être sélectionné dans le groupe comprenant Axol C62 (ADARA), Cutina CP (BASF), Cutina GMS (BASF), Emulium 22 (GATTEFOSSE), Emulium Delta (GATTEFOSSE), Emulium melifera (GATTEFOSSE), Eumulgin SG (BASF), Montanov 202 (Seppic), Montanov 68 (Seppic), Montanov 82 (Seppic), Montanov L (Seppic), Olivem 1000 (Univar), Olivoil avenate (ACS Phyto), Xyliance (MASSO). Le tensioactif peut être dérivé de sucres, tel qu'un polyglycoside d'alkyle, un ester de sorbitane, un ester de saccharose (ou sucroester), ou encore un alkylméthylglucamide. Par exemple, le tensioactif peut être un polyglycoside d'alkyle, tel qu'un alkylpolyglucoside. Les polyglycosides d'alkyle sont une classe de tensioactifs non ioniques largement utilisés dans diverses applications cosmétiques, domestiques et industrielles. Biodégradables, ces tensioactifs sont généralement des dérivés du glucose et des alcools gras. Le tensioactif dérivé de sucre peut également être un sucroester d'acides gras, ou ester de saccharose d'acide gras, tel que par exemple E473. Les sucroesters d'acides gras sont listés au Codex Alimentarius comme émulsifiants et stabilisants. Entre autres usages, les sucroesters sont insérés dans des glaces, des confiseries, des chewing-gums, des spiritueux, des compléments alimentaires, des préparations pour nourrissons et enfants en bas âge, ainsi que comme traitement surfacique de fruits frais. Hormis l'alimentation transformée, des sucroesters peuvent être insérés dans les produits cosmétiques et les pharmaceutiques, e.g. en tant qu'émulsifiants pour crèmes, lotions nettoyantes, lotions et pommades. Le tensioactif dérivé de sucre peut également être un ester de sorbitane, tel que E491 (Monostéarate de sorbitane, Span 60), E492 (Tristéarate de sorbitane, Span 65), E493 (Monolaurate de sorbitane, Span 20), E494 (Monooléate de sorbitane, Span 80), E495 (Monopalmitate de sorbitane, Span 40), E496 (Trioléate de sorbitane, Span 85), E432 (Monolaurate de polyoxyéthylène sorbitane, polysorbate 20), E433 (Monooléate de polyoxyéthylène sorbitane, polysorbate 80), E434 (Monopalmitate de polyoxyéthylène sorbitane, polysorbate 40), E435 (Monostéarate de polyoxyéthylène sorbitane, polysorbate 60), E436 (Tristéarate de polyoxyéthylène sorbitane, polysorbate 65). Le tensioactif dérivé de sucres peut être sélectionné dans le groupe comprenant le Simulsol AS 48, Simulsol SL 7G, Coco Glucoside, Decyl Glucoside, Lauryl Glucoside, Sodium Lauryl Glucose Carboxylate.

Le tensioactif peut également être dérivé d'acide-aminés, tel que le Sodium Cocoyl Glutamate, Disodium Cocoyl Glutamate, Sodium Lauroyl Glutamate, dérivé de peptide, tel que le Sodium Cocoyl Protéine Hydrolysée de Blé, le Sodium Cocoyl Collagène Hyrolysé. Le tensioactif peut être un alkyl PEG sulfosuccinate tel que le Disodium Laureth Sulfosuccinate, Disodium Deceth Sulfosuccinate, une acylsarcosine tel que le Sodium Lauroyl Sarcosinate Sodium Cocoyl Sarcosinate, un acyl iséthionate tel que le Sodium Cocoyl Iséthionate. Le tensioactif peut être le Disodium Laureth Sufosuccinate, Cocamidopropyl Bétaïne, Coco Bétaïne, Sodium Coco Sulfate, Sodium Lauryl Sulfoacétate. Le tensioactif peut être un glycolipide tels que les rhamnolipides (RL), mannosylerythritol lipides (MEL), trehalipides (TL), xylolipides, sophorolipides, un lipopeptide tels que la fengycine, l'iturine, la surfactine.

Selon une ou plusieurs formes de réalisation, l'au moins un tensioactif est sélectionné dans le groupe comprenant les dendrimères, tels que, par exemple, ceux de type Dendri-TAC décrits dans la demande PCT/IB2016/052952, les oligomères de type F*ᵢ*TAC*ₙ* ou H*ᵢ*TAC*ₙ*, le TPGS 1000, le TPGS 750M, et combinaisons de ceux-ci. Pour chaque tensioactif, la concentration en tensioactif peut par exemple être adaptée à la concentration en composé lipophile en vue d'obtenir des émulsions à faible indice de polydispersité, et influer ainsi sur le rendement d'extraction et/ou la pureté en substance d'intérêt. Selon une ou plusieurs formes de réalisation, la concentration en tensioactif dans le mélange excluant la source est comprise entre 0,5 mg/mL et 250 mg/mL, par exemple entre 5 mg/mL et 250 mg/mL. Au-delà de la limite supérieure de cette plage de valeurs, le rendement d'extraction de substance(s) d'intérêt peut diminuer. Cette diminution peut résulter d'une plus grande viscosité entraînant des difficultés de transfert des substances d'intérêt de la source vers la phase lipidique discontinue de l'émulsion.

Selon une ou plusieurs formes de réalisation, l'émulsification est effectuée en maintenant la température du mélange entre environ 0°C et environ 60°C, par exemple entre 4°C et 60°C.

Selon une ou plusieurs formes de réalisation, la température de l'émulsion ou de la formulation ne dépasse pas 60°C lors du procédé. En particulier pour les substances d'intérêt susceptibles de se dégrader rapidement en présence d'air et/ou lorsqu'elles subissent un traitement thermique, le procédé selon ces formes de réalisation apporte donc une valeur ajoutée considérable dans les secteurs cosmétique, agroalimentaire et/ou pharmaceutique. Selon une ou plusieurs formes de réalisation, la température du mélange ne dépasse pas 25°C durant l'émulsification, et reste par exemple comprise entre 0°C et 20°C, par exemple entre 5°C et 15°C.

Selon une ou plusieurs formes de réalisation, l'émulsification peut s'effectuer par le biais d'un bain à ultrasons et/ou d'une tige à ultrasons (sonotrodre) et/ou d'un réacteur ultrasonore de type « cup-horn » et/ou d'un disperseur-homogénéiseur (e.g. de type Ultraturrax^{®}). Selon une ou plusieurs formes de réalisation, le mélange est agité durant l'émulsification. Selon une ou plusieurs formes de réalisation, la durée d'émulsification est comprise entre environ 0,5 seconde et environ 5 heures. Selon une ou plusieurs formes de réalisation, la durée d'émulsification ne dépasse pas 2 heures, et est par exemple comprise entre 0,5 heure et 1,5 heures.

Selon une ou plusieurs formes de réalisation du procédé dans lequel l'émulsification du mélange comprend la sonification du mélange, la sonification s'effectue à une puissance comprise entre environ 100 W et environ 800 W, à une fréquence comprise entre environ 20 kHz et environ 2000 kHz, en mode pulsé ou continu.

Selon une ou plusieurs formes de réalisation, la phase lipidique discontinue se présente sous forme de gouttelettes de diamètre moyen (D) prédéterminé. Selon une ou plusieurs formes de réalisation, le diamètre moyen (D) prédéterminé desdites gouttelettes est compris entre environ 30 nm et environ 6000 nm. Pour certaines applications des émulsions obtenues par le procédé selon la présente description, le diamètre moyen (D) des gouttelettes de la phase lipidique est préférentiellement compris dans une plage de valeurs prédéterminées. Par exemple, les émulsions utilisées en relation avec la biodisponibilité orale peuvent faire intervenir des phases lipidiques se présentant sous forme de gouttelettes dont le diamètre moyen (D) est préférentiellement inférieur à 200 nm.

En outre, selon une ou plusieurs formes de réalisation de la présente description l'indice de polydispersité (ou PdI) des émulsions peut être préférentiellement inférieur à 0,5, par exemple inférieur à 0,3, ou encore inférieur à 0,2. Pour certaines émulsions obtenues par le procédé selon une ou plusieurs formes de réalisation de la présente description, un indice de polydispersité supérieur à 0,3 peut traduire une moins bonne stabilité de l'émulsion, pouvant par exemple se refléter par une moins bonne tenue à la lyophilisation.

Le procédé selon la présente description comprend en outre l'ajout d'au moins un cryoprotecteur au mélange. Cet ajout a lieu une fois le mélange émulsifié. La concentration en cryoprotecteur dans le mélange excluant la source est comprise entre 50 mg/mL (ou 5 % p/v, lorsqu'exprimé en rapport poids-volume par rapport au volume total du mélange) et 100 mg/mL (ou 10 % p/v). L'ajout d'un cryoprotecteur peut notamment permettre de conférer une protection supplémentaire aux émulsions obtenues après émulsification, par exemple lors de l'étape de lyophilisation. Le cryoprotecteur peut d'une part remplacer les liaisons hydrogènes entre l'eau et le tensioactif, maintenant ainsi l'organisation spatiale de l'émulsion, et d'autre part peut diminuer les interactions entre les tensioactifs de différentes gouttelettes, génératrices d'agrégats, et ainsi éviter la déstabilisation des gouttelettes de l'émulsion. En outre, le cryoprotecteur peut former une matrice amorphe et permet d'éviter la formation des cristaux de glace, car ceux-ci peuvent être à l'origine de mécanismes de déstabilisation des gouttelettes.

Selon une ou plusieurs formes de réalisation l'au moins un cryoprotecteur est sélectionné dans le groupe comprenant les polymères, les acides aminés, les composés saccharidiques tels que les mono-, di- et polysaccharides, et combinaisons de ceux-ci. Selon une ou plusieurs formes de réalisation, l'au moins un cryoprotecteur est sélectionné dans le groupe comprenant le tréhalose, le saccharose, le maltose, le glucose, le mannitol, l'hydroxypropyl-β-cyclodextrine, et combinaisons de ceux-ci. Dans d'autres exemples de réalisation, l'au moins un cryoprotecteur est un polymère, tel qu'un polyvinylpyrrolidone ou un alcool polyvinylique. L'au moins un cryoprotecteur peut également être un acide aminé, tel que la glycine.

Selon une ou plusieurs formes de réalisation, la concentration de l'au moins un cryoprotecteur dans le mélange excluant la source est comprise entre 50 mg/mL (ou 5 % p/v) et 100 mg/mL (ou 10 % p/v), et sélectionnée en fonction de facteurs tels que la composition du système, la rapidité de refroidissement ainsi que la température de congélation. Selon le premier aspect de la présente description, l'ajout de l'au moins un cryoprotecteur se fait une fois le mélange émulsifié, dans un rapport poids-volume compris entre 5 % p/v et 10 % p/v par rapport au volume total du mélange excluant la source, par exemple compris entre 6 % p/v et 8 % p/v. L'ajout d'un cryoprotecteur dans de telles concentrations peut par exemple permettre d'augmenter l'homogénéité d'émulsions obtenues par un procédé d'extraction selon la présente description comprenant une étape de lyophilisation de l'émulsion suivie d'une étape de réhydratation.

Selon une ou plusieurs formes de réalisation, l'au moins un composé lipophile, l'au moins un tensioactif et l'au moins un cryoprotecteur sont biocompatibles. Par substance biocompatible, on fait référence à une substance sans effets toxiques ou nuisibles sur la santé et utilisable pour des applications cosmétique et/ou pharmaceutique et/ou alimentaire. Par exemple, selon une ou plusieurs formes de réalisations, l'au moins un tensioactif et l'au moins un cryoprotecteur peuvent être considérés inoffensifs selon les critères reconnus par l'USFDA (United States Food and Drug Administration) et bénéficier de l'appellation « GRAS » (pour Generally Recognized As Safe).

Selon une ou plusieurs formes de réalisation, le procédé comprend en outre une étape de traitement « post-extraction », i.e. une étape postérieure à l'émulsification du mélange conduisant à la formation d'une émulsion. Cette étape de traitement peut par exemple comprendre une sonification ultérieure de l'émulsion obtenue après émulsification du mélange : cette étape de traitement peut alors être réalisée, par exemple, par le biais d'une tige à ultrasons (sonotrodre). L'étape de traitement « post-extraction » peut également par exemple comprendre une ou plusieurs étapes d'ultra-centrifugation à des vitesses comprises entre 1000G et 50 000G. Selon une ou plusieurs formes de réalisation, cette étape de traitement peut permettre d'abaisser à la fois le diamètre moyen D des gouttelettes et l'indice de polydispersité de l'émulsion.

Selon une ou plusieurs formes de réalisation, le procédé comprend en outre une étape de prétraitement préalable à l'émulsification du mélange, comprenant la macération à chaud du mélange comprenant l'eau, la source, l'au moins un tensioactif, et l'au moins un composé lipophile, à une température comprise entre environ 20°C et environ 60°C. Selon une ou plusieurs formes de réalisation, l'étape de prétraitement peut également comprendre un broyage, un passage au disperseur-homogénéiseur (e.g. Ultraturrax^{®}), au bain à ultrasons, ou au four à microondes en fonction de la source à extraire.

Le procédé selon la présente description comprend en outre la lyophilisation de l'émulsion. Par exemple, l'étape de lyophilisation peut s'effectuer après émulsification et ajout d'au moins un cryoprotecteur. L'étape de lyophilisation de l'émulsion peut conduire à la formation d'une émulsion sous la forme d'une formulation sèche. Ainsi, en un minimum d'étapes, des formulations sèches enrichies en substances potentiellement bioactives et liposolubles, peuvent être obtenues. Les émulsions correspondantes sont faciles à reconstituer, par simple réhydratation, i.e. par ajout d'une certaine quantité d'eau ou solution aqueuse à la formulation sèche pour reformer une émulsion. L'émulsion reconstituée n'aura pas nécessairement les mêmes caractéristiques que l'émulsion de départ, i.e. l'émulsion avant lyophilisation. Par exemple, le diamètre moyen D des gouttelettes et/ou l'indice de polydispersité PdI de l'émulsion reconstituée peuvent différer sensiblement de leurs valeurs dans l'émulsion avant lyophilisation et après lyophilisation/réhydratation (i.e. lyophilisation suivie d'une réhydratation).Selon une ou plusieurs formes de réalisation, le procédé comprenant la lyophilisation de l'émulsion suivie d'une étape de réhydratation, comprend en outre une étape de traitement « post-réhydratation », i.e. une étape postérieure à la réhydratation d'une formulation sèche obtenue par lyophilisation. Cette étape peut par exemple consister en la sonification de l'émulsion obtenue après réhydratation, réalisée par exemple par le biais d'une tige à ultrasons (sonotrodre). Selon une ou plusieurs formes de réalisation, cette étape peut permettre d'abaisser à la fois le diamètre moyen D des gouttelettes et l'indice de polydispersité de l'émulsion obtenue après réhydratation.

L'étape de lyophilisation peut être adaptée à la nature de la substance d'intérêt et à sa sensibilité inhérente, ainsi qu'au volume de mélange après émulsification. La durée de l'étape de lyophilisation peut varier de quelques heures à plusieurs jours. Selon une ou plusieurs formes de réalisation, l'étape de lyophilisation comporte au moins trois sous-étapes : la congélation où le mélange après émulsification est amené à une température inférieure à environ -20°C ; la sublimation a lieu à une pression inférieure à environ 500 µbars afin de sublimer l'eau dudit mélange; et la dessiccation où ledit mélange résultant est amené à une température supérieure à environ 20°C afin de diminuer le taux d'humidité résiduelle.

Lors de l'étape de lyophilisation, l'au moins un cryoprotecteur peut conférer une stabilité à l'émulsion durant le traitement thermique qu'elle subit.

Selon un deuxième aspect ne faisant pas partie de l'objet revendiqué mais utile afin de comprendre l'invention, la présente description concerne une formulation sèche obtenue par le procédé selon le premier aspect.

Afin d'assurer la stabilité, la conservation et le stockage des émulsions produites par le procédé selon le premier aspect, la présente invention inclut leur transformation en formulations sèches, par lyophilisation des émulsions, afin de faciliter et d'étendre leur champ d'utilisation à des secteurs industriels variés. Lesdites formulations sèches, obtenues par lyophilisation des émulsions, permettent, parmi d'autres avantages, de limiter toute dégradation de type oxydation des substances d'intérêts qu'elles contiennent.

La présente description permet d'aboutir en un minimum d'étapes, par un procédé « one-pot », à des formulations sèches enrichies en substances potentiellement bioactives liposolubles et faciles à reconstituer par simple réhydratation. Outre leur intérêt en matière de stockage, de conservation et d'utilisation, ces formulations pourraient permettre d'améliorer la stabilité et la biodisponibilité de molécules bioactives, ce qui ouvre des perspectives d'applications dans des domaines variés, dont certains à haute valeur ajoutée, tels que les cosmétiques, l'agroalimentaire ou encore l'industrie pharmaceutique. Dans le cas d'extraits à visée agroalimentaire (e.g. les compléments ou additifs alimentaires) ou pharmaceutique, de telles formulations peuvent par exemple permettre une amélioration de la biodisponibilité orale des molécules peu hydrosolubles qu'elles contiennent.

Selon un aspect supplémentaire (ne faisant pas partie de l'objet revendiqué), la présente description concerne l'utilisation de la formulation sèche selon le deuxième aspect, dans la manufacture d'un produit contenant au moins une substance d'intérêt extraite d'une source comprenant l'au moins une substance d'intérêt ou au moins un précurseur de celle-ci, par ajout à ladite formulation sèche d'un composé sélectionné dans le groupe comprenant de l'eau ou une huile.

Selon un autre aspect (ne faisant pas partie de l'objet revendiqué), la présente description concerne l'utilisation de la formulation sèche selon le deuxième aspect dans les domaines des cosmétiques, de l'agroalimentaire, de la pharmaceutique, de la probiotique et/ou de la nutraceutrique.

Les formes de réalisation décrites ci-dessus ne sont pas exhaustives. Notamment, il est entendu que des formes de réalisation supplémentaires peuvent être envisagées sur la base de différentes combinaisons des formes de réalisation explicitement décrites. Sauf spécification contraire dans la présente description, il sera apparent pour l'homme du métier que toutes les formes de réalisation décrites ci-dessus peuvent être combinées entre elles. Par exemple, sauf spécification contraire, toutes les caractéristiques des formes de réalisation décrites ci-dessus, quelles que soient les formes de réalisation du procédé auxquelles elles se réfèrent, peuvent être combinées avec ou remplacées par d'autres caractéristiques d'autres formes de réalisation.

Des formes de réalisation selon les aspects référencés ci-dessus ainsi que des avantages supplémentaires apparaîtront à la lecture de la description détaillée suivante et des revendications annexées.

### Brève description des figures

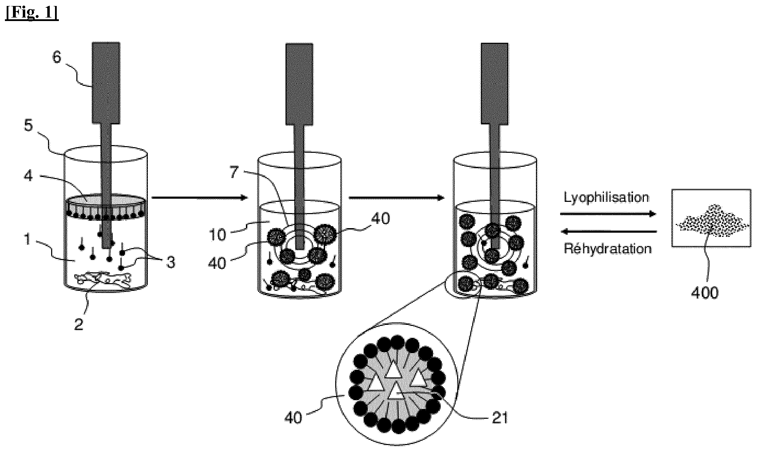

[Fig. 1], un schéma illustrant un exemple de procédé selon un mode de réalisation de l'invention.

### Description détaillée

Dans la description détaillée suivante des formes de réalisation de la présente invention, de nombreux détails spécifiques sont exposés afin de fournir une compréhension plus approfondie de la présente description. Cependant, il apparaîtra à l'homme du métier que la présente description peut être mise en oeuvre sans ces détails spécifiques. Dans d'autres cas, des caractéristiques bien connues n'ont pas été décrites en détail pour éviter de compliquer inutilement la description.

La FIG. 1 illustre une forme de réalisation du procédé d'extraction selon la présente description, comprenant en outre une étape supplémentaire de lyophilisation d'une émulsion obtenue par sonification. Le mélange à émulsifier comprend de l'eau (1), une source végétale (2), i.e. une racine de *Curcuma longa*, un tensioactif (3), un composé lipophile (4). Le mélange est contenu dans un récipient (5), et son émulsification comprend une étape de sonification réalisée par le biais d'une tige à ultrasons (6), capable d'émettre des ondes ultrasonores (7). La sonification du mélange conduit à la formation d'une émulsion comprenant une phase aqueuse continue (10) et une phase lipidique discontinue se présentant sous forme de gouttelettes (40) présentant une certaine teneur en substance d'intérêt (21), i.e. en l'occurrence, la curcumine. Un cryoprotecteur est ensuite ajouté au mélange, et l'étape de lyophilisation conduit à la formation d'une formulation sèche (400). L'ajout d'eau à cette formulation sèche, i.e. la réhydratation, peut permettre à la formulation sèche de se réhydrater et conduit à une émulsion présentant les mêmes caractéristiques qu'avant séchage. Cependant, dans d'autres formes de réalisation du procédé d'extraction selon la présente description que celle illustrée par la FIG. 1, la réhydratation de l'émulsion peut conduire à une émulsion présentant des caractéristiques différentes de l'émulsion avant séchage, e.g. en termes de diamètre moyen D et d'indice de polydispersité.

Des exemples de réalisation d'un procédé d'extraction selon la présente description sont détaillés ci-dessous, dans lesquels la source choisie est une racine de *Curcuma longa* afin d'en extraire la curcumine, la substance d'intérêt. La curcumine est un polyphénol dont les propriétés pharmacologiques variées (antiinflammatoire, antioxydant, anticancéreux, traitement de certaines maladies neurodégénératives....) ne sont à ce jour que partiellement explorées. La curcumine montre en outre une faible solubilité dans l'eau (11 ng/ml dans une solution tampon pH = 5), ce qui rend le procédé selon la présente description particulièrement attractif en vue d'extraire la curcumine de la *Curcuma longa.* Préalablement à leurs tentatives d'extraction de la curcumine, les inventeurs ont testé la solubilité de cette molécule dans différents composés lipophiles, par dosage HPLC. Parmi les composés lipophiles testés, la Tributyrine, notamment, s'est avérée être un bon candidat. De façon intéressante, ce composé lipophile est une huile biocompatible, ce qui peut le rendre attractif pour certaines applications du procédé de l'invention dans les domaines des cosmétiques, de l'agroalimentaire, de la pharmaceutique, de la nutraceutique et/ou de la probiotique.

Les émulsions numérotées 12 à 18, pour lesquelles certaines conditions caractéristiques du procédé permettant de les obtenir, pour le reste identiques, ont été rassemblées dans la Table 1, sont des émulsions obtenues par le procédé de l'invention selon autant d'exemples différents de formes de réalisation du procédé de l'invention. Ces formes de réalisation du procédé aboutissant aux émulsions 12 à 18 impliquent toutes l'utilisation du tensioactif TPGS 1000 (α-tocophéryl polyéthylène glycol 1000 succinate), à savoir un tensioactif commercial dérivé de la vitamine E (tocophérol), ainsi que l'utilisation de Tributyrine en tant qu'au moins un composé lipophile du mélange. Dans les émulsions 13 à 18, un deuxième composé lipophile a été rajouté dans le mélange fourni par le procédé, sous la forme d'une cire, i.e. cire de jasmin pour l'émulsion 13 et Suppocire NB pour l'émulsion 14, ou sous la forme d'une autre huile, i.e. les Triglycérides C8-C10 pour l'émulsion 18.

Comme apparent pour l'émulsion 14, les inventeurs ont réussi à obtenir, via le procédé de l'invention, une émulsion contenant une teneur en curcumine correspondante à un taux d'extraction de 100%. Ce taux d'extraction, fixé à 100% dans l'acétone pour un montage d'extraction au Soxhlet pendant 8 heures, a été obtenu selon les conditions des inventeurs en seulement 2 minutes de sonification du mélange conduisant à la formation de l'émulsion 14.

**[Table 1]**

| Emulsion | 12 | 13 | 14 | 18 |
|---|---|---|---|---|
| m _{Tributyrine} (mg) | 206 | 181 | 175 | 85 |
| m _{Triglycérides C8-C10} (mg) | 0 | 0 | 0 | 15 |
| m _{SuppocireNB} (mg) | 0 | 0 | 25 | 0 |
| m _{cire de jasmin} (mg) | 0 | 25 | 0 | 0 |
| m _{Curcuma Longa} (mg) | 100 | 100 | 100 | 100 |
| m _{TPGS 1000} (mg) | 60,8 | 60,8 | 60,8 | 60,8 |
| m _{H20} (mg) | 2027,6 | 2027,6 | 2027,6 | 2027,6 |
| D (nm) | 286,5 | 168,3 | 194,7 | 113 |
| PdI | 0,296 | 0,232 | 0,254 | 0,257 |
| Taux d'extraction (%) | 67 | 88 | 100 | 93 |

En outre, il semble que le remplacement d'une quantité du premier composé, *i.e.*, la Tributyrine, par une quantité environ égale à son équivalent massique en cire de jasmin (émulsion 13) ou en SuppocireNB (émulsion 14) permette d'améliorer le taux d'extraction et de réduire l'indice de polydispersité PdI, en comparaison de l'émulsion 12 obtenue seulement à partir de Tributyrine.

Certains détails protocolaires de réalisation et d'analyse des émulsions 12 à 18 sont fournis dans les paragraphes ci-dessous.

### Protocole de réalisation des émulsions 12 à 18 :

Le composé lipophile seul (*Tributyrine*) ou les deux composés lipophiles (*Tributyrine*/*Triglycérides C8-C10 ou Tributyrine*/*cire de jasmin ou Tributyrinel Suppocire NB)* a été font été pesé(s), avec un certain ratio dans le cas du mélange de deux composés lipophiles. En outre, dans le cas d'un mélange huile/cire, le mélange huile/cire a été placé dans un bain à 40°C jusqu'à obtention d'un mélange homogène. La phase aqueuse a été préparée en dispersant un tensioactif (*TPGS 1000*) dans de l'eau milliQ^{®}. La phase aqueuse a ensuite été ajoutée à l'au moins un composé lipophile et le tout a été agité pendant 20 secondes à l'aide d'un vortex. Cette solution a ensuite été versée dans un tube à centrifuger conique de 50 mL dans lequel une certaine masse de *Curcuma longa* a préalablement été pesée (100 mg).

Une émulsion grossière a été préparée en émulsifiant l'au moins un composé lipophile, la phase aqueuse et la source végétale premièrement à l'aide d'un vortex pendant 10 secondes puis en plaçant le tube à centrifuger au bain à ultrasons pendant 5 minutes à température ambiante.

A partir de cette émulsion grossière, une émulsion plus fine a ensuite été préparée grâce à un ultrasonicateur (BIOBLOCK SCIENTIFIC, Vibracell 7504). La sonde à ultrasons (Φ=13 mm) a été placée dans le tube à centrifuger pendant 16,75 minutes (en mode pulsé, correspondant à 2 minutes de sonification au total) dans un bain de glace. La température du mélange pendant la sonification du mélange a été mesurée et comprise dans une plage d'environ [4°C - 20°C]. Le rapport cyclique appliqué a été de 11.94% et l'intensité de sonification de 60% (450W).

Après avoir retiré la sonde à ultrasons de l'émulsion, la même sonde a été trempée dans un tube contenant 2 mL d'eau milliQ^{®}. L'eau de rinçage a ensuite été ajoutée à l'émulsion et le tout a été centrifugé 1 minute à 1100G.

L'émulsion résultante peut ensuite être diluée puis aliquotée en lots de 2 mL. 300 µL d'une solution aqueuse de cryoprotecteur (concentration 500 mg/mL), e.g. tréhalose ou maltose, peuvent alors y être ajoutés. Ces aliquots peuvent ensuite être placés au congélateur une nuit puis lyophilisés pendant une journée.

### Protocole d'analyse des émulsions 12 à 18 :

Le surnageant obtenu après centrifugation a été récupéré et la distribution en taille des gouttes a été analysée par diffusion dynamique de la lumière (DLS) grâce à un Nanosizer Nano-S (Malvern Instrument). Le surnageant, dilué par 10 dans l'eau milliQ^{®}, a été placé dans une cuve en quartz de 45 µL et a subi 10 mesures de 10 secondes chacune. Le diamètre hydrodynamique ou diamètre moyen D a été obtenu en moyennant les résultats des 10 mesures. Les mesures ont été réalisées à un angle de 173° en utilisant un laser dont la longueur d'onde est de 633 nm. Les données de la DLS sont calculées sur une base en intensité.

La concentration en curcumine du surnageant a été dosée par chromatographie liquide haute performance après avoir dilué l'émulsion dans de l'acétonitrile puis filtré sur filtre seringue 0,2 µm nylon.

Un système Shimadzu avec un détecteur à barrette de diodes (SPD-M20A) a été équipé d'un système de pompage (LC-20 AD), d'un dégazeur (DGU-20A3), d'un module de communication (CBM-20A) et d'un four pour colonne (Waters). La séparation a été réalisée sur une colonne Phenomenex Kinetex Biphenyl (100 Å, 4,6*100 mm, 2,6 µm) avec une température de colonne fixée à 30°C. L'analyse des données a été réalisée avec le logiciel LabSolution. Un gradient linéaire de A (eau contenant 0,1% d'acide trifluoroacétique) et B (acétonitrile contenant 0,1% d'acide trifluoraoacétique) a été utilisé avec un gradient d'élution comme suit (v/v) : 0 min, B 37% ; 10 min, B 50% ; 15min, B 100% maintenu pendant 10 min. Le débit a été de 1,3 mL/min et le volume d'injection de 5 µl. La détection a été obtenue à 420 nm.

Les Tables 2 à 9 suivantes rassemblent certaines caractéristiques d'émulsions, i.e. les émulsions 19 à 65, préparées selon une ou plusieurs formes de réalisation du procédé de l'invention. Ces émulsions ont été préparées selon des protocoles de réalisation non détaillés mais similaires à ceux décrits plus haut pour la préparation des émulsions 12 à 18. Le protocole d'analyse des émulsions 19 à 65 est en revanche identique à celui utilisé pour les émulsions 12 à 18.

Chaque Table 2 à 9 vise à présenter des résultats comparables car obtenus toutes conditions égales par ailleurs.

**[Table 2]**

| Emulsion | 19 | 18 | 20 | 22 |
|---|---|---|---|---|
| m _{Tributyrine} (mg) | 100 | 85 | 85 | 42,5 |
| m _{Triglycérides C8-C10} (mg) | 0 | 15 | 0 | 0 |
| m _{SuppocireNB} (mg) | 0 | 0 | 15 | 7,5 |
| m _{Curcuma Longa} (mg) | 100 | 100 | 100 | 100 |
| m _{TPGS 1000} (mg) | 60,8 | 60,8 | 60,8 | 60,8 |
| m _{H20} (mg) | 2027,6 | 2027,6 | 2027,6 | 2027,6 |
| D (nm) | - | 113 | 109 | - |
| PdI | > 0,3 | 0,257 | 0,251 | > 0,3 |
| Taux d'extraction (%) | 74 | 93 | 87 | 82 |

La Table 2 montre des émulsions 19 et 20 réalisées en pesant la même masse totale de composé(s) lipophile(s) que l'émulsion 18 du Tableau 1, à savoir 100 mg. Les résultats montrent notamment le bénéfice de l'ajout d'un second composé lipophile en vue de réduire le PdI. L'émulsion 22 correspond à l'émulsion 20 pour laquelle la masse de composé lipophile a été diminuée de moitié, i.e. 50 mg, au détriment de l'homogénéité de l'émulsion, dont le PdI est supérieur à 0,3.

Les inventeurs ont également montré que l'émulsion 18, lorsque lyophilisée en présence de 6,5 % (p/v) de tréhalose (utilisé en tant que cryoprotecteur), puis réhydratée, présente un D de 147 nm et un PdI de 0,26 (résultat ne figurant pas dans la Table 2).

**[Table 3]**

| Emulsion | 23 | 24 | 25 | 26 | 27 |
|---|---|---|---|---|---|
| m _{Tributyrine} (mg) | 100 | 100 | 100 | 100 | 100 |
| m _{Curcuma Longa} (mg) | 100 | 150 | 200 | 250 | 300 |
| m _{TPGS 1000} (mg) | 60,8 | 60,8 | 60,8 | 60,8 | 60,8 |
| V _{H20} (mL) | 4000 | 4000 | 4000 | 4000 | 4000 |
| D (nm) | - | 134 | 112 | 111 | 119 |
| PdI | > 0,3 | 0,278 | 0,234 | 0,209 | 0,182 |
| Taux d'extraction (%) | 87 | 90 | 87 | 94 | 97 |

La Table 3 indique que le taux d'extraction augmente avec l'accroissement de la quantité de source (*Curcuma longa*) comprise dans le mélange, tout comme l'homogénéité des émulsions, ce qui se traduit pour cette dernière par une diminution du PdI.

**[Table 4]**

| Emulsion | 27 | 28 | 29 | 30 | 31 |
|---|---|---|---|---|---|
| m _{Tributyrine} (mg) | 100 | 100 | 100 | 100 | 100 |
| m _{Curcuma Longa} (mg) | 300 | 300 | 300 | 300 | 300 |
| m _{TPGS 1000} (mg) | 60,8 | 60,8 | 60,8 | 60,8 | 60,8 |
| V _{H20} (mL) | 4000 | 4000 | 4000 | 4000 | 4000 |
| Post-extraction (Centrifugation) | 1100G 1mn | 1100G 1mn + 5000G 5mn | 1100G 1mn + 10000G 5mn | 1100G 1mn + 15000G 5mn | 1100G 1mn + 20000G 5mn |
| D (nm) | 119 | 116 | 116 | 115 | 113 |
| PdI | 0,182 | 0,127 | 0,116 | 0,112 | 0,114 |
| Taux d'extraction (%) | 97 | 81 | 77 | 83 | 81 |

Contrairement à l'émulsion 27, opaque, les émulsions 28 à 31 présentées dans la Table 4 sont translucides, du fait de l'inclusion dans le procédé d'extraction dont elles sont issues d'une étape de traitement « post-extraction » comprenant plusieurs étapes d'ultra-centrifugation.

L'addition d'une deuxième étape d'ultra-centrifugation dans l'étape de post-traitement se traduit par une légère baisse du taux d'extraction, mais également par une augmentation de l'homogénéité des émulsions reflétée par la baisse du PdI.

**[Table 5]**

| Emulsion | 32 | 33 | 34 | 35 | 36 |
|---|---|---|---|---|---|
| CG1 | Tributyrine | Capryol90 | Squalène | Tocophérol | Triglycérid es (C8-C10) |
| m _{CG1} (mg) | 100 | 100 | 100 | 100 | 100 |
| m _{Curcuma lonea} (mg) | 100 | 100 | 100 | 100 | 100 |
| m _{TPGS 1000} (mg) | 60,8 | 60,8 | 60,8 | 60,8 | 60,8 |
| m _{H20} (mg) | 2027,6 | 2027,6 | 2027,6 | 2027,6 | 2027,6 |
| Prétraiteme nt | UltraTurrax ^{®} | UltraTurrax ^{®} | UltraTurrax ^{®} | UltraTurrax ^{®} | UltraTurrax ^{®} |
| D (nm) | - | - | 138 | 199 | - |
| PdI | > 0,3 | > 0,3 | 0,230 | 0,277 | > 0,3 |
| Taux d' extraction (%) | 76 | 80 | 79 | 83 | 81 |

La Table 5 montre l'influence de la nature du composé lipophile dans le cadre d'émulsions comprenant un seul composé lipophile (ou Corps Gras 1, CG1).

La série d'émulsions 32 à 36 présentées dans la Table 5 montre notamment la bonne homogénéité des émulsions contenant le Tocophérol (également appelé Vitamine E) ou le Squalène en tant que composés lipophiles.

**[Table 6]**

| Emulsion | 35 | 37 | 38 | 39 |
|---|---|---|---|---|
| CG1 | Tocophérol | Tocophérol | Tocophérol | Tocophérol |
| m _{CG1} (mg) | 100 | 100 | 85 | 85 |
| m _{Squalène} (mg) | 0 | 0 | 15 | 0 |
| m _{Tributyrine} (mg) | 0 | 0 | 0 | 15 |
| m _{Curcuma longa} (mg) | 100 | 100 | 100 | 100 |
| m _{TPGS 1000} (mg) | 60,8 | 60,8 | 60,8 | 60,8 |
| m _{H20} (mg) | 2027,6 | 2027,6 | 2027,6 | 2027,6 |
| Prétraitement | UltraTurrax^{®} | - | - | UltraTurrax^{®} |
| D (nm) | 199 | - | 136 | 133 |
| PdI | 0,277 | > 0,3 | 0,226 | 0,245 |
| Taux d'extraction (%) | 83 | 76 | 79 | 71 |

La Table 6 montre l'influence d'une étape de prétraitement spécifique, ainsi que l'influence de l'ajout d'un deuxième composé lipophile dans le cadre d'émulsions comprenant du tocophérol en tant que composé lipophile (ou Corps Gras 1, CG1). Les résultats montrent que l'étape de prétraitement préalable comprenant un passage du mélange au disperseur-homogénéiseur (de type UltraTurrax^{®}), permet d'abaisser le PdI des émulsions. Le même effet est observé lorsqu'un deuxième composé lipophile est utilisé, en comparaison du cas d'un composé lipophile unique.

**[Table 7]**

| Emulsion | 40 | 41 | 42 |
|---|---|---|---|
| Emulsification | Ultrasons 2mn | UltraTurrax^{®} 16mn | UltraTurrax^{®} 2mn |
| m _{Tributyrine} (mg) | 255 | 255 | 255 |
| m _{Triglycérides C8-C10} (mg) | 45 | 45 | 45 |
| m _{Curcuma Longa} (mg) | 300 | 300 | 300 |
| m _{TPGS 1000} (mg) | 180 | 180 | 180 |
| V _{H20} (mL) | 6000 | 6000 | 6000 |
| D (nm) | 88 | 134 | 121 |
| PdI | 0,260 | 0,230 | 0,240 |
| Taux d'extraction (%) | 77 | 83 | 83 |

La Table 7 permet de comparer une émulsion obtenue par un procédé comprenant une étape d'émulsification du mélange par sonification (émulsion 40) avec deux émulsions obtenues par un procédé comprenant une étape d'émulsification par passage au disperseur-homogénéiseur (de type UltraTurrax^{®}).

Le temps total de sonification de 2mn pour l'émulsion 40, indiqué dans la Table 7, correspond au temps total d'application des ultrasons au mélange. De même, pour les émulsions 41 et 42, le temps indiqué correspond au temps total où le disperseur-homogénéiseur est appliqué au mélange.

Le taux d'extraction et l'homogénéité des émulsions 41 et 42 (obtenue avec le disperseur-homogénéiseur) sont similaires et légèrement plus élevés que les valeurs correspondantes caractérisant l'émulsion 40 (obtenue par sonification).

Les inventeurs ont également montré que lorsque les émulsions subissent une étape de traitement « post-extraction » consistant en une ultracentrifugation à 5000G pendant 5mn, les valeurs de (D ; PdI) pour les émulsions 40 et 42 sont, respectivement, de (81 ; 0,200) et (111 ; 0,222) (résultat ne figurant pas dans la Table 7).

Les Tables 8 et 9 ci-dessous présentent les caractéristiques d'autres émulsions obtenues par des procédés d'extraction selon la présenté description, s'appliquant à d'autres sources que la racine de *Curcuma longa*, telles que la fleur de lavande (Table 8) ou l'écorce d'orange (Table 9). Dans les deux cas, la source est végétale et contient une huile essentielle représentant la substance d'intérêt.

Deux différents tensioactifs ont été utilisés pour extraire l'huile essentielle de lavande, à savoir le TPGS 1000 pour les émulsions 43 à 52 et le H₁₂TAC₅ pour les émulsions 53 à 55. Les émulsions 43 à 47, ne contenant pas d'huile de tournesol, illustrent également la possibilité que l'au moins un composé lipophile soit exclusivement contenu dans la source, comme le prévoit la présente description selon une ou plusieurs formes de réalisation du procédé. La Table 8 illustre en outre l'utilisation d'un cryoprotecteur (le tréhalose) en différentes quantités en vue d'abaisser l'indice de polydispersité des émulsions après lyophilisation/réhydratation.

Les émulsions 56 à 60, ne contenant pas d'huile de tournesol, illustrent la possibilité que l'au moins un composé lipophile soit exclusivement contenu dans la source, comme le prévoit la présente description selon une ou plusieurs formes de réalisation du procédé. La Table 9 illustre en outre l'utilisation d'un cryoprotecteur (le tréhalose) en différentes quantités en vue d'abaisser l'indice de polydispersité des émulsions après lyophilisation/réhydratation

**[Table 8]**

| Emulsion | Conditions d' analyse | m Huile de Tournesol (mg) | m Lavande (g) | TPGS 1000 (g/L) | H₁₂TAC₅ (g/L) | V _{H20} (mL) | Tréhalose (% p/v) | D (nm) | PdI |
|---|---|---|---|---|---|---|---|---|---|
| 43 | Avant | 0 | 2 | 0,66 | 0 | 15 | 0 | 120 | 0,226 |
| 44 | Après | 0 | 2 | 0,66 | 0 | 15 | 0 | 252 | 0,399 |
| 45 | Après | 0 | 2 | 0,66 | 0 | 15 | 6,5 | 173 | 0,247 |
| 46 | Après | 0 | 2 | 0,66 | 0 | 15 | 10 | 167 | 0,243 |
| 47 | Après | 0 | 2 | 0,66 | 0 | 15 | 15 | 152 | 0,216 |
| 48 | Avant | 10 | 1,25 | 1 | 0 | 10 | 0 | 106 | 0,206 |
| 49 | Après | 10 | 1,25 | 1 | 0 | 10 | 0 | 338 | 0,551 |
| 50 | Après | 10 | 1,25 | 1 | 0 | 10 | 6,5 | 172 | 0,177 |
| 51 | Après | 10 | 1,25 | 1 | 0 | 10 | 10 | 177 | 0,163 |
| 52 | Après | 10 | 1,25 | 1 | 0 | 10 | 15 | 152 | 0,173 |
| 53 | Avant | 10 | 1,25 | 0 | 1 | 10 | 0 | 132 | 0,209 |
| 54 | Après | 10 | 1,25 | 0 | 1 | 10 | 0 | 460 | 0,545 |
| 55 | Après | 10 | 1,25 | 0 | 1 | 10 | 6,5 | 137 | 0,218 |

**[Table 9]**

| Emulsion | Conditions d' analyse | m _{Huile de Tournesol} (mg) | m _{Ecorce d'Orange} (g) | TPGS 1000 (g/L) | V _{H20} (mL) | Tréhalose (% p/v) | D (nm) | PdI |
|---|---|---|---|---|---|---|---|---|
| 56 | Avant | 0 | 2 | 1 | 10 | 0 | 137 | 0,160 |
| 57 | Après | 0 | 2 | 1 | 10 | 0 | 160 | 0,462 |
| 58 | Après | 0 | 2 | 1 | 10 | 6,5 | 146 | 0,362 |
| 59 | Après | 0 | 2 | 1 | 10 | 10 | 159 | 0,426 |
| 60 | Après | 0 | 2 | 1 | 10 | 15 | 152 | 0,343 |
| 61 | Avant | 10 | 2 | 1 | 10 | 0 | 137 | 0,201 |
| 62 | Après | 10 | 2 | 1 | 10 | 0 | 140 | 0,914 |
| 63 | Après | 10 | 2 | 1 | 10 | 6,5 | 126 | 0,970 |
| 64 | Après | 10 | 2 | 1 | 10 | 10 | 233 | 0,734 |
| 65 | Après | 10 | 2 | 1 | 10 | 15 | 131 | 0,967 |

## Revendications

1. Procédé d'extraction d'au moins une substance d'intérêt à partir d'une source, le procédé comprenant les étapes suivantes :
- la fourniture d'un mélange comprenant de l'eau, une source végétale, animale, minérale, procaryote, et/ou eucaryote unicellulaire comprenant l'au moins une substance d'intérêt ou au moins un précurseur de celle-ci, au moins un tensioactif, et au moins un composé lipophile;
- l'émulsification du mélange, conduisant à la formation d'une émulsion ; dans lequel l'émulsion comprend :
- une phase aqueuse continue; et
- une phase lipidique discontinue comprenant l'au moins un composé lipophile, l'au moins un tensioactif et l'au moins une substance d'intérêt extraite de la source durant l'émulsification ;
dans lequel le procédé comprend en outre l'ajout d'au moins un cryoprotecteur au mélange et la lyophilisation de l'émulsion ; et
dans lequel l'ajout d'au moins un cryoprotecteur au mélange est effectué après l'émulsification du mélange, dans un rapport poids-volume compris entre 5 % p/v et 10 % p/v par rapport au volume total du mélange excluant la source.

2. Procédé selon la revendication 1, dans lequel l'émulsification du mélange comprend la sonification du mélange.

3. Procédé selon la revendication 1 ou la revendication 2, dans lequel la source est végétale.

4. Procédé selon l'une quelconque des revendications précédentes, dans lequel l'au moins un tensioactif est sélectionné dans le groupe comprenant les dendrimères de type Dendri-TAC, les oligomères de type F*ᵢ*TAC*ₙ* ou H*ᵢ*TAC*ₙ*, le TPGS 1000, le TPGS 750M, les tensioactifs dérivés de sucres et/ou d'acide-aminés, et combinaisons de ceux-ci.

5. Procédé selon l'une quelconque des revendications précédentes, dans lequel l'émulsification est effectuée en maintenant la température du mélange entre environ 4°C et environ 60 °C**.**

6. Procédé selon l'une quelconque des revendications précédentes, dans lequel la durée de l'émulsification est comprise entre environ 0,5 seconde et environ 5 heures.

7. Procédé selon l'une quelconque des revendications précédentes, dans lequel ladite phase lipidique discontinue se présente sous forme de gouttelettes de diamètre moyen D prédéterminé.

8. Procédé selon l'une quelconque des revendications précédentes, dans lequel l'au moins un cryoprotecteur est sélectionné dans le groupe comprenant les polymères, les acides aminés, les composés saccharidiques tels que les mono-, di- et polysaccharides, et combinaisons de ceux-ci.

9. Procédé selon l'une quelconque des revendications précédentes, dans lequel l'au moins un composé lipophile, l'au moins un tensioactif et l'au moins un cryoprotecteur sont biocompatibles.

10. Procédé selon l'une quelconque des revendications précédentes, comprenant en outre une étape de prétraitement préalable à l'émulsification du mélange, dans lequel l'étape de prétraitement comprend la macération du mélange à une température comprise entre environ 20°C et environ 60°C.

## Patentansprüche

1. Verfahren zur Extraktion mindestens einer Substanz von Interesse aus einer Quelle, wobei das Verfahren die folgenden Schritte aufweist:
- Bereitstellung einer Mischung, die Wasser, eine pflanzliche, tierische, mineralische, prokaryotische und/oder eukaryotische einzellige Quelle aufweist, die die mindestens eine Substanz von Interesse oder mindestens eine Vorstufe davon, mindestens ein Tensid und mindestens eine lipophile Verbindung enthält;
- Emulgieren der Mischung, wodurch eine Emulsion gebildet wird;
wobei die Emulsion aufweist:
- eine kontinuierliche Wasserphase; und
- eine diskontinuierliche Lipidphase, die die mindestens eine lipophile Verbindung, das mindestens eine Tensid und die mindestens eine Substanz von Interesse aufweist, die während der Emulgierung aus der Quelle extrahiert wird;
wobei das Verfahren ferner die Zugabe von mindestens einem Kryoschutzmittel zu der Mischung und die Gefriertrocknung der Emulsion aufweist; und
wobei die Zugabe des mindestens einen Kryoschutzmittels zu der Mischung nach der Emulgierung der Mischung in einem Gewicht-Volumen-Verhältnis zwischen 5 % Gew./Vol. und 10 % Gew./Vol. bezogen auf das Gesamtvolumen der Mischung unter Ausschluss der Quelle durchgeführt wird.

2. Verfahren nach Anspruch 1, wobei die Emulgierung der Mischung die Beschallung der Mischung aufweist.

3. Verfahren nach Anspruch 1 oder 2, wobei die Quelle pflanzlich ist.

4. Verfahren nach einem der vorhergehenden Ansprüche, wobei das mindestens eine Tensid aus der Gruppe ausgewählt ist, die Dendrimere vom Typ Dendri-TAC, Oligomere vom Typ FᵢTACₙ oder HᵢTACₙ, TPGS 1000, TPGS 750M, von Zucker und/oder Aminosäuren abgeleitete Tenside und Kombinationen davon aufweist.

5. Verfahren nach einem der vorhergehenden Ansprüche, wobei die Emulgierung durchgeführt wird, indem die Temperatur der Mischung zwischen etwa 4 °C und etwa 60 °C gehalten wird.

6. Verfahren nach einem der vorhergehenden Ansprüche, wobei die Dauer des Emulgierens zwischen etwa 0,5 Sekunden und etwa 5 Stunden beträgt.

7. Verfahren nach einem der vorhergehenden Ansprüche, wobei die diskontinuierliche Lipidphase in Form von Tröpfchen mit einem vorbestimmten mittleren Durchmesser D vorliegt.

8. Verfahren nach einem der vorhergehenden Ansprüche, wobei das mindestens eine Kryoschutzmittel ausgewählt ist aus der Gruppe bestehend aus Polymeren, Aminosäuren, Saccharidverbindungen wie Mono-, Di- und Polysacchariden und Kombinationen davon.

9. Verfahren nach einem der vorhergehenden Ansprüche, wobei die mindestens eine lipophile Verbindung, das mindestens eine Tensid und das mindestens eine Kryoschutzmittel biokompatibel sind.

10. Verfahren nach einem der vorhergehenden Ansprüche, ferner aufweisend einen Vorbehandlungsschritt vor dem Emulgieren der Mischung, wobei der Vorbehandlungsschritt das Mazerieren der Mischung bei einer Temperatur zwischen etwa 20°C und etwa 60°C aufweist.

## Claims

1. A process for extracting at least one substance of interest from a source, the process comprising the following steps:
- providing a mixture comprising water, a plant, animal, mineral, prokaryote, and/or unicellular eukaryote source comprising the at least one substance of interest or at least one precursor thereof, at least one surfactant, and at least one lipophilic compound;
- emulsifying the mixture, leading to the formation of an emulsion;
wherein the emulsion comprises:
- a continuous aqueous phase; and
- a discontinuous lipid phase comprising the at least one lipophilic compound, the at least one surfactant, and the at least one substance of interest extracted from the source during emulsification;
wherein the process further comprises adding at least one cryoprotectant to the mixture and freeze-drying the emulsion; and
wherein adding at least one cryoprotectant to the mixture is carried out after emulsifying the mixture, in a weight-to-volume ratio comprised between 5% w/v and 10% w/v based on the total volume of the mixture excluding the source.

2. The process according to claim 1, wherein emulsifying the mixture comprises sonicating the mixture.

3. The process according to claim 1 or claim 2, wherein the source is a plant source.

4. The process according to any one of the preceding claims, wherein the at least one surfactant is selected from the group comprising Dendri-TAC type dendrimers, F*ᵢ*TAC*ₙ* or H*ᵢ*TAC*ₙ* type oligomers, TPGS 1000, TPGS 750M, sugar- and/or amino acid-derived surfactants, and combinations thereof.

5. The process according to any one of the preceding claims, wherein emulsifying is performed by maintaining the temperature of the mixture between about 4°C and about 60°C.

6. The process according to any one of the preceding claims, wherein the duration of emulsifying is comprised between about 0.5 seconds and about 5 hours.

7. The process according to any one of the preceding claims, wherein said discontinuous lipid phase is in the form of droplets of predetermined mean diameter D.

8. The process according to any one of the preceding claims, wherein the at least one cryoprotectant is selected from the group comprising polymers, amino acids, saccharide compounds such as mono-, di- and polysaccharides, and combinations thereof.

9. The process according to any one of the preceding claims, wherein the at least one lipophilic compound, the at least one surfactant, and the at least one cryoprotectant are biocompatible.

10. The process according to any one of the preceding claims, further comprising a pretreatment step prior to emulsifying the mixture, wherein the pretreatment step comprises macerating the mixture at a temperature comprised between about 20°C and about 60°C.
